# EUROPEAN PATENT APPLICATION

(11) **EP 1 480 115 A2**
(43) Date of publication of application: **24.11.2004**
(21) Application number: 03258198.5
(22) Date of filing: 23.12.2003
(51) Int. Cl.: G06F 3/023

(54) **Character string input assistance program and apparatus and method for inputting character string**

(30) Priority: 21.05.2003 JP 2003143200
(71) Applicant: IT Coordinate, Inc, Tokyo 105-0004 (JP)
(72) Inventor: Kinoshita, Masayoshi, Minato-ku Tokyo 105-0004 (JP); Kuribara, Masaru, Minato-ku Tokyo 105-0004 (JP)
(74) Representative: Maury, Richard Philip

(57) **Abstract**

A character string input assistance program of the present invention comprises a step (S01) of receiving an input unconverted character string; a step (S02) of receiving a conversion command for the unconverted character string; a step (S03, S04) of executing display for prompting selection of a plurality of conversion candidate databases constituted by pre-storing conversion candidate character strings in accordance with the conversion command; a step (S05) of receiving a command for selecting a conversion candidate database; a step (S07) of referring to the conversion candidate database selected in accordance with the command for selecting the conversion candidate database to present a conversion candidate character string included in the conversion candidate database; a step (S08) of receiving a command for selecting a conversion candidate character string; and a step (S09) of supplying the conversion candidate character string selected in accordance with the command for selecting the conversion candidate character string to the other predetermined program.

## Description

### BACKGROUND OF THE INVENTION

The present invention relates to an input assistance program of character strings in a computer or the like, and an apparatus and a method for inputting character strings.

### PRIOR ART

In medical institutions, in order to make more efficient and share medical record management, electronic medical charting by using an information processor such as a computer has been promoted in place of a conventional medical record (chart) which uses paper (e.g., see Patent Document 1).

In such an electronic medical chart system, efficient sharing of data requires, for example, standardization of descriptions of disease names. There has conventionally been a case in which even the same disease is recorded by using different disease names among doctors. This has resulted in a loss of convenience of data sharing. Thus, use of disease names, disease name codes etc., defined in International Statistical Classification of Diseases and Related Health Problems (abbreviated to "ICD", hereinafter) prepared by World Health Organization (WHO) has been proposed to standardize contents described in electronic medical charts among the medical institutions.

Health, Labor and Welfare Ministry of Japan has distributed disease name master retrieval software which a user such as a doctor uses to refer to a disease name master defined in the ICD when he prepares an electronic medical chart (e.g., see Nonpatent Document 1). Use of such a disease name master retrieval software enables input of an accurate and standardized disease name in the electronic medical chart by so-called cutting and pasting of the retrieved disease name.

### [Patent Document 1]

Japanese Patent Application Laid-Open No. 2001-344342

### [Nonpatent Document 1]

"Disease Name Master Retrieval Software 'Byoumeikun' ", by K. HATANO and three others, [online], [retrieved on April 25 of 2003], Internet <URL: http://www.jcmi2002.med.kyushu-u.ac.jp/jcmi-kakunin/JCMI22/H-15/paper.html>

Incidentally, in the promotion of electronic medical charting, there has been a request for easy input of standardized accurate disease names, disease codes etc. However, the aforementioned conventional disease name master retrieval software enables only reference to the disease names etc. Input of the retrieved disease name in the electronic medical chart or the like must go through a complex process of cutting and pasting, etc.

### SUMMARY OF THE INVENTION

The present invention has been made to solve the foregoing problem, and its object is to facilitate accurate input of a standardized character string.

A character string input assistance program of the present invention comprises a step of receiving an input unconverted character string; a step of receiving a conversion command for the unconverted character string; a step of executing display for prompting selection of a plurality of conversion candidate databases constituted by prestoring conversion candidate character strings in accordance with the conversion command; a step of receiving a command for selecting a conversion candidate database; a step of referring to the conversion candidate database selected in accordance with the command for selecting the conversion candidate database to present a conversion candidate character string included in the conversion candidate database; a step of receiving a command for selecting a conversion candidate character string; and a step of supplying the conversion candidate character string selected in accordance with the command for selecting the conversion candidate character string to another predetermined program.

According to the present invention, the plurality of conversion candidate databases can be selected by the operation of converting the character string, and the conversion candidate character string included in the selected conversion database is input to the other program only by the selection operation. Therefore, standardized character strings can be accurately and easily input by preparing the conversion candidate databases which prestore the standardized character strings.

The conversion candidate database which stores, e.g., the standardized character strings, is not referred to unless a user further selects a conversion candidate database after he inputs a character string and a selection command. Thus, in the case of general character string conversion, only normal dictionary software is referred to, and a processing load on an apparatus can be reduced.

In this case, the other predetermined program may be, e.g., an operating system (OS: basic software) for a computer, a portable telephone terminal or the like. Alternatively, it may be an application program (application software) for executing specific processing on the OS. The application program may be an electronic medical chart preparation program, and the plurality of conversion candidate databases may include at least one selected from a disease name database, a disease name code database and a medicine name database.

The character string input assistance program may be installed integrally with the OS or the application program. However, processing may be carried out cooperatively with an input method (IM) program constituted independently of the other predetermined program. That is, the assistance program can be used with a plurality of other programs for general purposes by executing processing at a front end processor (FEP) stage, which is convenient.

The character string input assistance program may be installed in a stand-alone apparatus to be used. Additionally, it may be installed in a server connected to a network such as Internet to be used as an application service provider (ASP).

Furthermore, a character string inputting apparatus of the present invention comprises a memory section for storing a plurality of conversion candidate databases constituted by prestoring conversion candidate character strings; an information processing section connected to the memory section; an input section for receiving an operation from a user; and a display section for presenting information to the user; the apparatus having functions of executing display for prompting input of a command for selecting a conversion candidate database on a display section during execution of another predetermined program in accordance with inputs of an unconverted character string and a conversion command, referring to the conversion candidate database selected in accordance with the input of the command for selecting the conversion candidate database to execute, on the display section, display for prompting input of a command for selecting a conversion candidate character string included in the conversion candidate database, and supplying the conversion candidate character string selected in accordance with the input of the command for selecting the conversion candidate character string to the other predetermined program.

### BRIEF DESCRIPTION OF THE DRAWINGS

FIG. 1 is a block diagram showing a constitution of a computer which comprises an embodiment of a character string conversion program of the present invention;
FIG. 2 is a flowchart showing an operation of a character string input assistance program in the computer of FIG. 1;
FIG. 3 is a view of an example of screen displaying in the computer of FIG. 1, showing a state in which a user inputs a character string to be converted;
FIG. 4 is a view of another example of screen displaying in the computer of FIG. 1, showing a screen display which includes a list of conversion candidate databases;
FIG. 5 is a view of another example of screen displaying in the computer of FIG. 1, showing a state in which a conversion candidate character string is displayed;
FIG. 6 is a view of another example of screen displaying in the computer of FIG. 1, showing a state in which an established character string is provided to an application program;
FIG. 7 is a view of another example of screen displaying in the computer of FIG. 1, showing a state in which an established character string is provided to an application program;
FIG. 8 is a view of yet another example of screen displaying in the computer of FIG. 1, showing a screen display of an application program which uses a re-input function;
FIG. 9 is a table showing a part of a Japanese standard product classification table used as a conversion candidate database of the present invention;
FIG. 10 is a view of a part of the Japanese standard product classification table used as the conversion candidate database of the present invention, showing an example included below that of FIG. 9;
FIG. 11 is a view showing an example of a medicine code (medicine group name database) according to another embodiment of the present invention;
FIG. 12 is a view showing an example of a medicine group name (component database) according to the embodiment of FIG. 11;
FIG. 13 is a view showing an example of a medicine group name (database of product name, common name, component name) according to the embodiment of FIG. 11; and
FIG. 14 is a view of an example of screen displaying according to the embodiment of FIG. 11, including a list of conversion candidate databases which have hierarchical structures.

### DESCRIPTION OF THE PREFERRED EMBODIMENTS

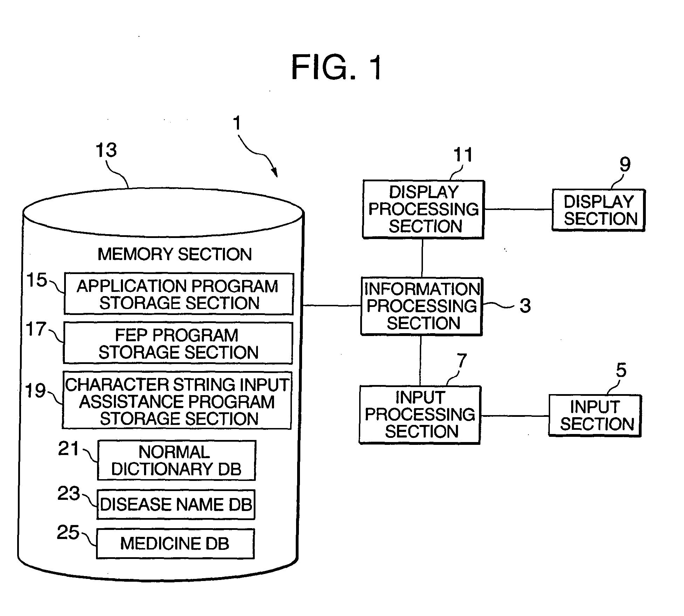

Next, description will be made of a computer to which a character string converter of the present invention is applied, and a character string conversion program installed therein. FIG. 1 is a block diagram showing a constitution of a computer of an embodiment. As shown in FIG. 1, a computer 1 comprises an information processing section 3 having a central processing unit (CPU), an input section 5 having a pointing device (e.g., keyboard or mouse) for receiving an operation from a user, and an input processing unit 7 which is an interface disposed between the input section 5 and the information processing section 3. The computer 1 further comprises a display section 9 having a display for presenting information to the user, and a display processing section 11 which is an interface disposed between a control section 1 and the display section 9.

A memory section 13 which comprises a memory such as a hard disk drive (HDD) or an optical reading/writing device is connected to the control section 1. In the memory section 13, an application program storage section 15 is disposed to store an application program such as an electronic medical chart preparation program, a Japanese or foreign language word processor program, a spreadsheet program or a database program. In the memory section 13, a front end processor (FEP) program storage section 17 is disposed to store an FEP program having a so-called input method (IM) function of converting a character string input by the user from the input section 5. Further, in the memory section 13, a character string input assistance program 19 is disposed to store a character string input assistance program.

Additionally, in the memory section 13, a normal dictionary database (DB) 21 is disposed to be referred to at the time of normal character string input. In the memory section 13, a disease name database (DB) 23 and a medicine name database (DB) 25 are disposed respectively to store standardized disease names and disease name codes, and standardized medicine names in association with related disease names.

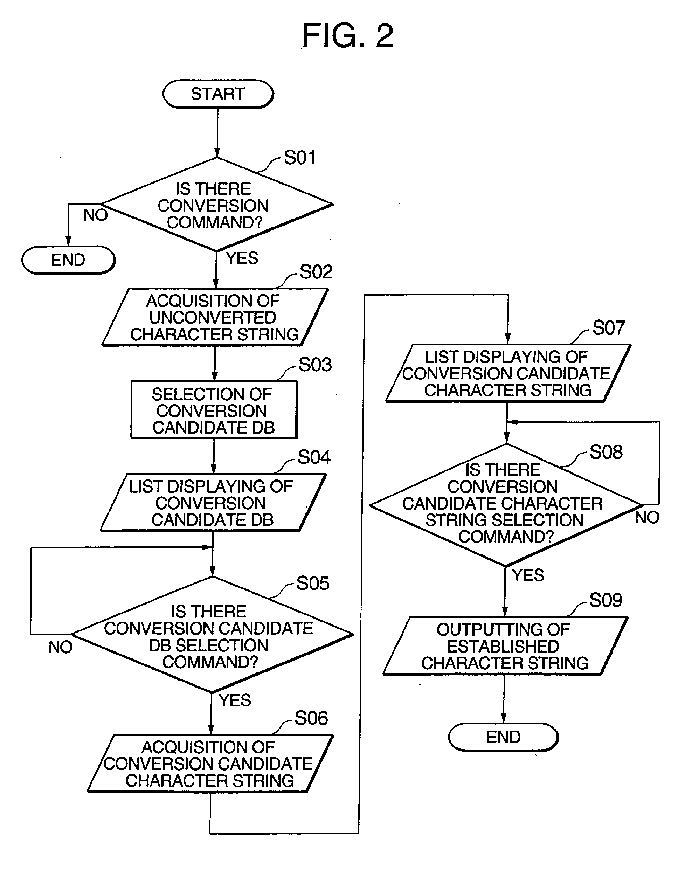

Next, description will be made of an operation of the character string input assistance program of the embodiment. FIG. 2 is a flowchart showing the operation of the character string input assistance program of the embodiment.

As shown in FIG. 2, the character string input assistance program comprises a step S01 of determining whether there is a conversion command input from the user or not, a step S02 of acquiring an unconverted character string, a step S03 of selecting a conversion candidate DB to be presented to the user, a step S04 of displaying a list of conversion candidate DB's, a step S05 of determining whether there is a command input from the user for selecting the conversion candidate DB or not, a step S06 of acquiring a conversion candidate character string from the selected conversion candidate DB, a step S07 of displaying a list of conversion candidate character strings, a step S08 of determining whether there is a command input for selecting the conversion candidate character string or not, and a step S09 of outputting the established character string. Hereinafter, description will be made in sequence of steps.

### (Step S01)

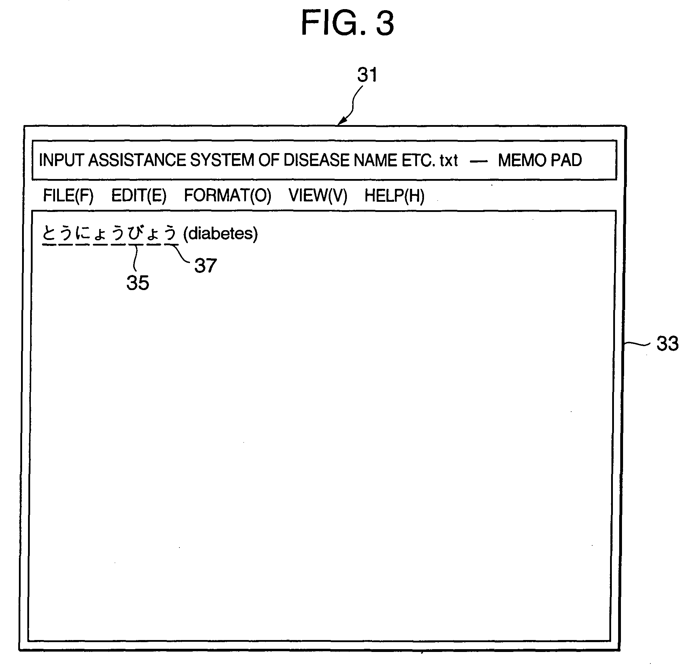

First, in a state in which an application program is executed to request input of a character string, the information processing section 1 waits until the user inputs a character string to be converted and a conversion command from the input section 5. FIG. 3 is a view showing an example of screen displaying when the user inputs the character string to be converted. As shown in FIG. 3, a character string input column 33 is disposed on a screen display 31. Text data or character string information such as a language (Japanese or a foreign language) or a sequence is input to the character string input column 33. The user can input the character string to be converted to the character string input column 33 through the input section 5. In FIG. 3, for example, a character string 35 to be converted which is "tonyobyo (diabetes)" in Romanized Japanese is input to the character string input column 33. Below the character string 35, a broken line 37 is displayed to indicate an unconverted state of the character string. Then, when the user inputs the conversion command from the input section 5, the process proceeds to step S02. The input of the conversion command is carried out by, for example, a predetermined operation of depressing a conversion key of the keyboard or the like.

### (Step S02)

After the input of the conversion command from the user, the information processing section 3 acquires a character string to be converted by the conversion command, and then proceeds to next step S03.

### (Step S03)

Then, for the character string to be converted, the information processing section 3 determines whether there is related information or not in the disease name DB 23 and the medicine name DB 25 which are conversion candidate DB's, and decides to present the DB's to the user if there is related information. For example, since the character string "tonyobyo (diabetes)" in Romanized Japanese to be converted is equivalent to "tonyobyo (diabetes)" which is a disease name (Romanized Japanese) stored in the disease name DB 23, the disease name DB 23 is presented to the user. Since there is medicine information related to "tonyobyo (diabetes)" stored in Romanized Japanese in the medicine name DB 25, the medicine name DB 25 is also presented to the user.

### (Step S04)

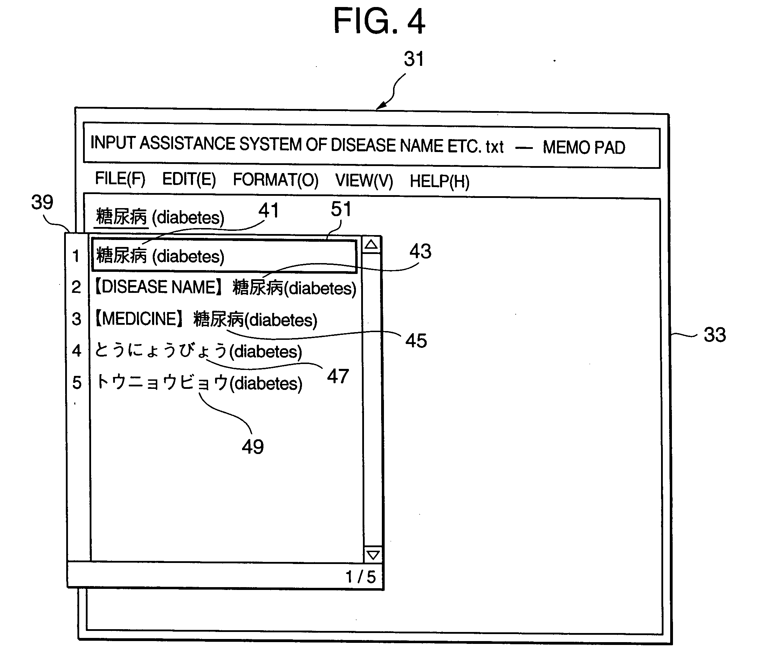

Then, the information processing section 3 displays a list of the conversion candidate DB's decided to be presented to the user in step S03 on the display section 9. FIG. 4 is a view showing an example of screen displaying which includes the list of the conversion candidate DB's. As shown in FIG. 4, the list of the conversion candidate DB's is displayed in a display area 39 similar to that for a list of conversion candidate character strings retrieved from a general dictionary in normal FEP processing. For example, in the case of FIG. 4, in the display area 39, there are a display 41 of "tonyobyo (diabetes)" in Romanized Japanese which is a conversion candidate character string retrieved from the general dictionary, a display 43 of "[disease name] tonyobyo (diabetes)" which indicates readiness to present the conversion candidate character string included in the disease name DB 23 regarding "tonyobyo (diabetes)", and a display 45 of "[medicine] tonyobyo (diabetes)" which indicates readiness to present the conversion candidate character string included in the medicine DB 25 regarding "tonyobyo (diabetes)". Additionally, in the display area 39, there are included a display 47 of a character string of "tonyobyo (diabetes)" in Romanized Japanese which indicates no execution of conversion processing for the character string to be converted, and a display 49 of a character string converted into "tonyobyo (diabetes)" of Romanized Japanese. A cursor 51 is superimposed on the display of the conversion candidate character string or the conversion candidate DB currently selected in the display area 39. For example, in the case of FIG. 4, the cursor 51 is displayed on the display 41.

### (Step S05)

Then, the information processing section 3 determines where there is a command input by the user from the input section 5 for selecting a conversion candidate DB or not. The input of the command for selecting the conversion candidate DB (disease name DB and medicine name DB in this case) by carrying out a predetermined operation such as a key operation or a pointing device operation to move the cursor 51 in the display area 39, and a predetermined operation such as key depressing.

### (Step S06)

Then, after the input of the command for selecting the conversion candidate DB, the information processing section 3 accesses the selected conversion candidate DB to acquire a conversion candidate character string. For example, if the user moves the cursor 51 to the display 43 to select the disease name DB 23, a character string regarding diabetes is acquired as a conversion candidate character string from the character strings stored in the disease name DB 23.

### (Step S07)

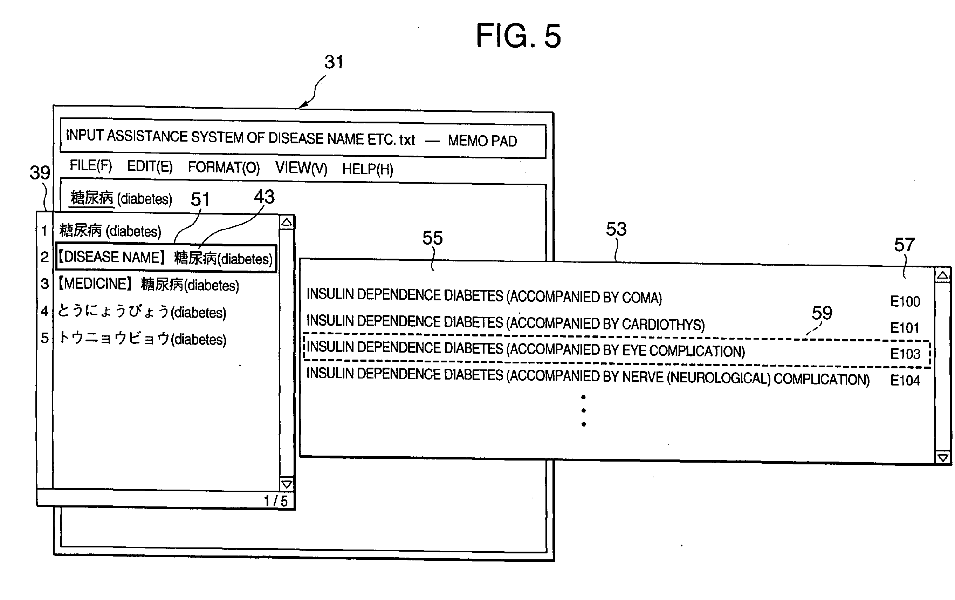

The information processing section 3 displays a display column 53 of the conversion candidate character string acquired in step S06 on the right side of the display area 39 of the display section 9. FIG. 5 shows an example of a screen display in which conversion candidate character strings are displayed. In the case of FIG. 5, for example, the display column 53 is constituted as a table of two horizontal rows. There is a display 55 of a standardized disease name on a left column, and there is a display 57 of a disease name code corresponding to the standardized disease name on a right column. Then, a number of disease names can be displayed by scrolling the screen in a longitudinal direction.

For example, in the case of FIG. 5, insulin dependence diabetes (accompanied by coma), insulin dependence diabetes (accompanied by cardiothys), insulin dependence diabetes (accompanied by eye complication) and insulin dependence diabetes (accompanied by nerve (neurological) complication) are displayed as disease names, and their corresponding disease name codes E100, E101, E103 and E104 are displayed. Additionally, a selection inhibition flag can be set to a specific included in the disease name DB 23. For example, in the case of FIG. 5, a selection inhibition flag "0" is set to the "insulin dependence diabetes (accompanied by eye complication) E103, and selection permission flags "1" are set to the other items. In this case, in the display column 53, the item "insulin dependence diabetes (accompanied by eye complication) E103" is a display 59 in which a display mode is different, e.g., reduced saturation of a display color, displaying of a specific color with a frame or the like, whereby selection inhibition is notified to the user. A display inhibition flag "0" can be set to a specified conversion candidate character string included in the disease name DB. In this case, display permission flags "1" are set to the other items, and only the items in which the display permission flags are set are displayed in the display column 53.

### (Step S08)

Then, the information processing section 3 determines whether there is a command input for selecting a conversion candidate character string or not. The input of the command for selecting the conversion candidate character string is carried out, for example, by the user who operates the cursor 61 displayed in the display column 53 to move to a desired item, and executes a predetermined operation such as depressing of a predetermined key.

### (Step S09)

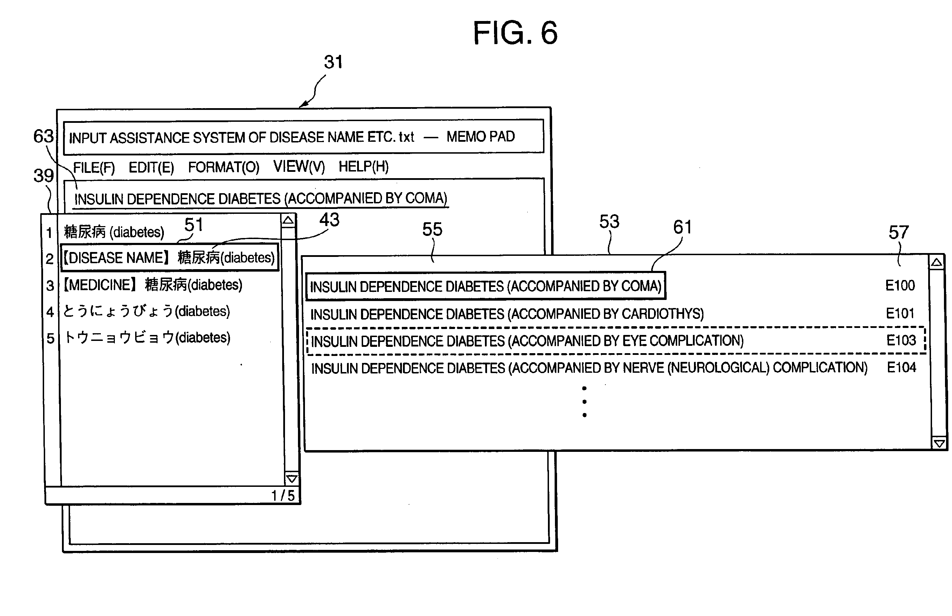

Then, after the input of the command for selecting the conversion candidate character string, the information processing section 3 outputs the selected conversion candidate character string as an established character string to the application program or the FEP program. At this time, the character string input assistance program provides the character string as text data or an argument to the application program or the FEP program. FIG. 6 shows an example of screen displaying in a state in which an established character string is provided to the application program. In the case of FIG. 6, the cursor 61 is superimposed on a display of a disease name "insulin dependence diabetes (accompanied by coma)". Then, the user inputs a command for selecting a conversion candidate character string in this state, whereby the character string "insulin dependence diabetes (accompanied by coma)" is input to be displayed in the character input column 33 of the application program.

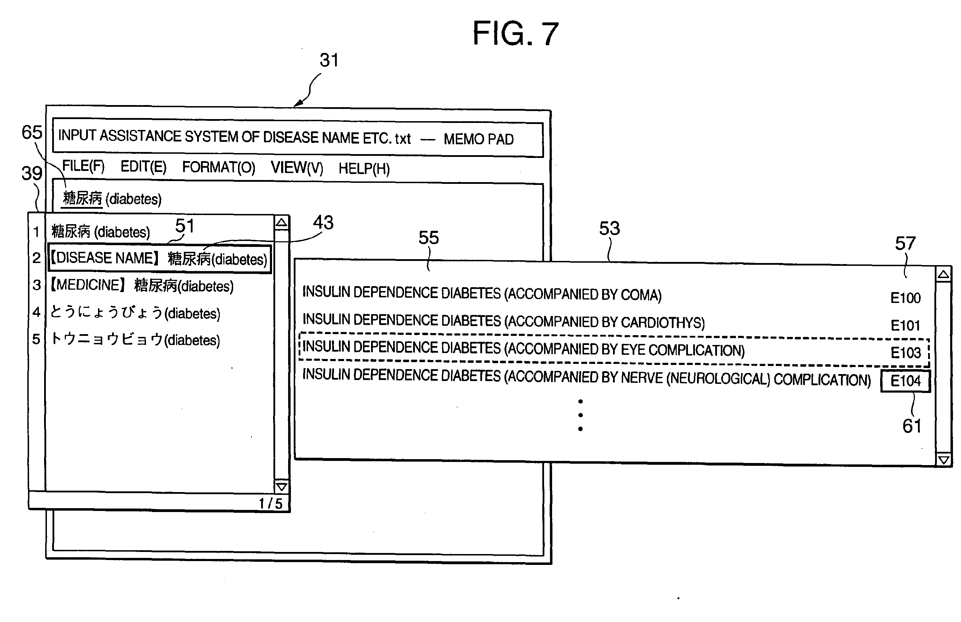

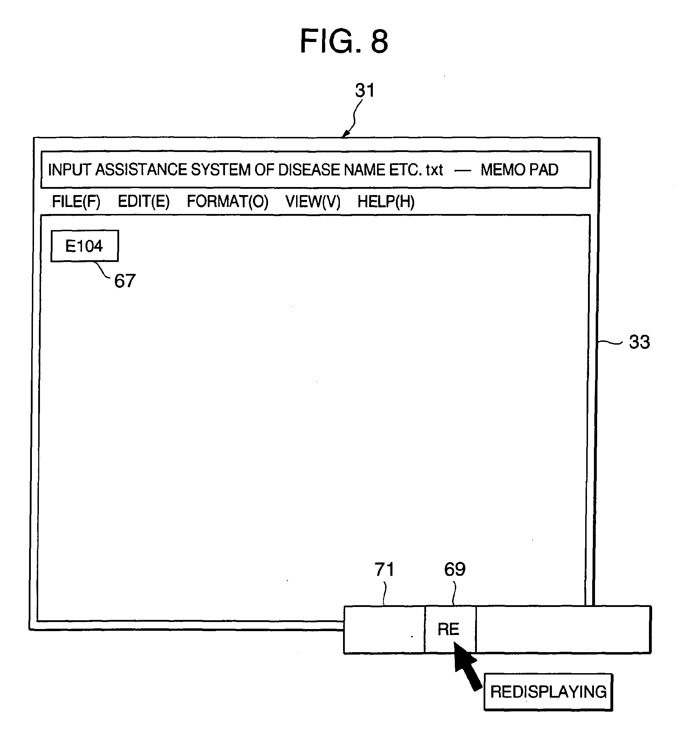

FIG. 7 is a view showing another example of screen displaying in a state in which an established character string is provided to the application program. In the case of FIG. 7, the cursor 61 is superimposed on a display of a disease name code "E104". Then, the user inputs a command for selecting a conversion candidate character string in this state, whereby a character string 67 of "E104" (see FIG. 8) is input to be displayed in the character input column 33 of the application program. Then, the displays are erased from the display area 39 and the display column 53 by a predetermined operation such as a predetermined key operation.

As it is constituted to work cooperatively with the FEP which is processing before the application program, the character string input assistance program of the embodiment can be used for general purposes in a plurality of application programs. For example, it can be used not only in the electronic medical chart preparation program but also various application programs for a word processor, a spreadsheet, presentation etc. It is also constituted not to influence the operation of the existing FEP at the time of installation or noninstallation.

Furthermore, according to the character string input assistance program of the embodiment, if the FEP program has a function of acquiring a character string established in the application program to execute conversion processing again, the established character string can be used as a key to call a conversion candidate display screen at the time of selecting the character string (re-input function). FIG. 8 is a view showing an example of screen displaying of an application program in which the re-input function is used. For example, the character string 67 "E104" input as the established character string is specified to input a predetermined re-input command in the character string input column 33 of the application program. Then, a list of conversion candidate character strings displayed at the time of conversion is displayed to return the screen to, e.g., the state shown in FIG. 7. In other words, a selection state immediately before is re-displayed. The re-input command is input by, for example, using the pointing device to select a re-input button display 69 on the screen, and executing a predetermined operation such as key depressing. According to the embodiment, the re-input button display 69 is disposed in an input method status bar 71 which can always be displayed during the operation of the computer.

As described above, according to the embodiment, the conversion candidate database such as a disease name DB can be selected by the conversion operation of the character string, and the character strings such as a disease name and a disease name code can be input to the electronic medical chart preparation program only by the selection operation. Thus, it is possible to accurately and easily input standardized disease names or the like.

The conversion candidate DB such as a disease name DB is not referred to unless the user selects the DB. Thus, in general character string conversion, only the general dictionary is referred to, whereby a processing load on the apparatus can be reduced.

The character string input assistance program is processed at the FEP stage which is processing before the application program. Thus, it can be used together with a plurality of other application programs for general purposes.

According to the embodiment, the conversion candidate character string databases are, e.g., a disease name DB and a medicine DB, and the application program is for medical purposes such as an electronic medical chart preparation program. However, the present invention is in no way limited to these.

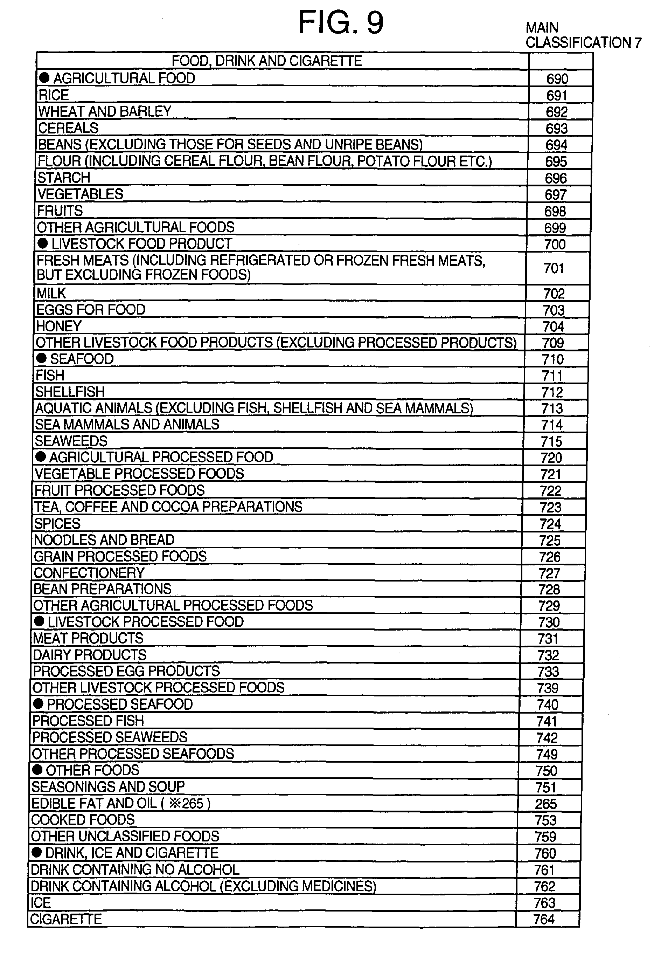

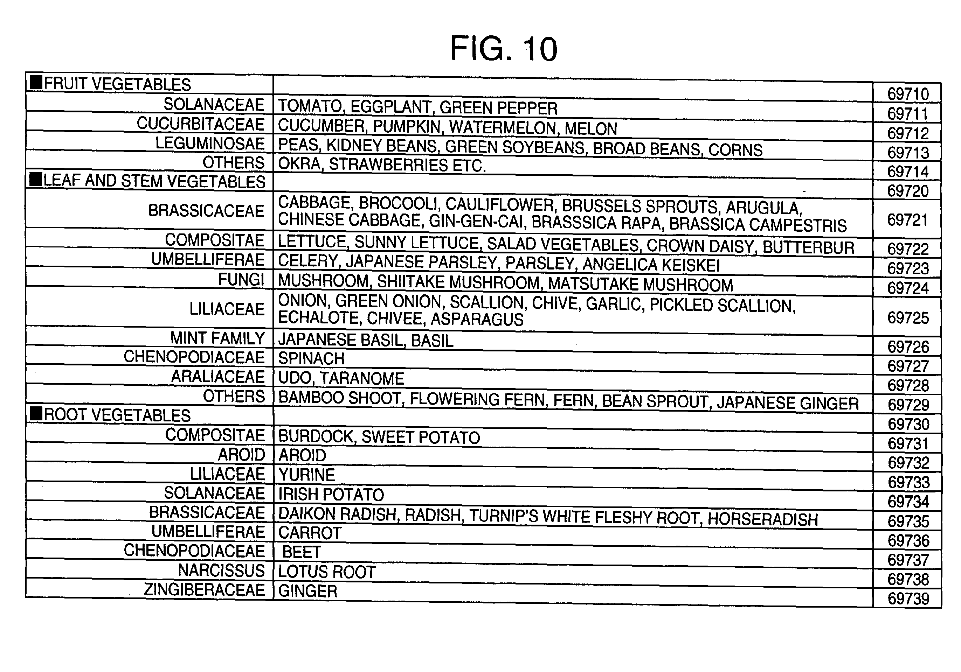

As an example, the invention can be applied to product statistics. For example, FIG. 9 is a table which is a part of Japanese Standard Product Classification defined by Bureau of Statistics at Ministry of Public Management, Home Affairs, Posts and Telecommunications, regarding foods, drinks and cigarettes. As shown in FIG. 9, for example, "agricultural food" is included in "food, drink and cigarette" belonging to a main classification 7, in which for example "rice", "vegetable" etc., are included. FIG. 10 is a table which is an example of items below the table of FIG. 9, regarding fruit vegetables, leaf and stem vegetables, and root vegetables.

As shown in FIG. 10, for example, there are family names of "solanaceae", "cucurbitaceae" etc., in "fruit vegetables". For example, "solanaceae" includes items "tomato, eggplant, green pepper". For example, a code number such as "69710" is attached to "solanaceae".

Assuming the character string input assistance program used together with a product statistical program or the like, if databases similar to those shown in FIGS. 9 and 10 are used as conversion candidate databases, for example, items such as "agricultural food" defined in FIG. 9 are input as unconverted character strings. Further, if a conversion command is input, it is possible to present conversion candidate databases regarding middle classification included in the main classification, e.g., "rice", "vegetables" etc., and to select desired classification items. In this case, for example, a constitution may be employed in which if the user selects a conversion candidate database regarding "vegetables", for example, a family name, an item name or its code name below "vegetables" similar to those shown in FIG. 10 can be selected as a conversion candidate character string. Then, when the user selects an item name or a code name, the item name or the code name is input to the input column of the application program.

The conversion candidate character string database may comprise two or more layers. That is, a constitution may be employed in which when an unconverted character string is input, selection of an optional database group from database groups each including a plurality of conversion candidate character strings is prompted, and then selection of a conversion candidate database included in the selected database group is prompted.

For example, if the character string input assistance program is used when a medicine name is input, the following constitution may be employed.

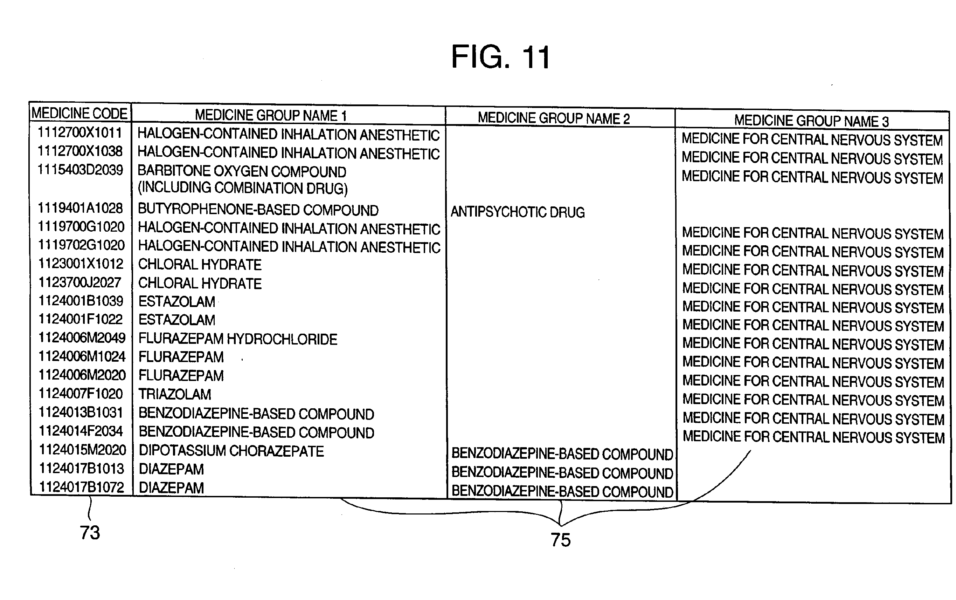

According to the constitution, in the memory section 13, for example, a database which is similar to that shown in FIG. 11 and which comprises medicine codes and medicine group names is disposed. This database is constituted as a table which includes a plurality of medicine codes 73 and medicine group names 75 corresponding to the medicine codes. Additionally, a plurality of medicine group names are correlated in accordance with kinds of medicine codes. For example, for a medicine code "1124017B1013", two medicine group names "diazepam" and "benzoazepine-based compound" are correlated to be stored.

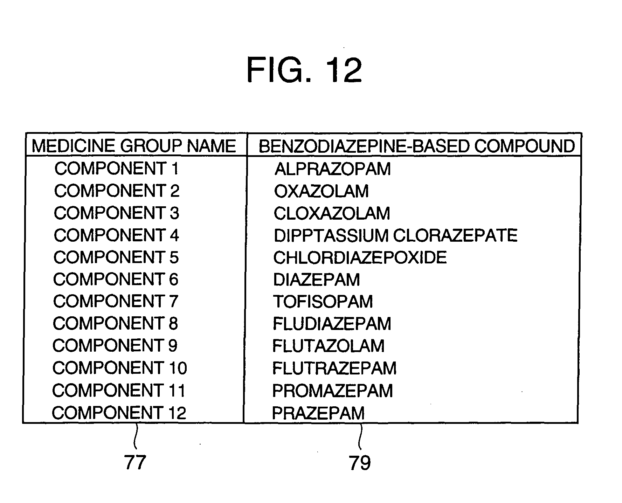

For example, as shown in FIG. 12, a database which comprises medicine group names and components may be disposed in the memory section 13. In this database, a predetermined medicine group name 77 and a plurality of component names 79 included in the medicine group are correlated to be stored.

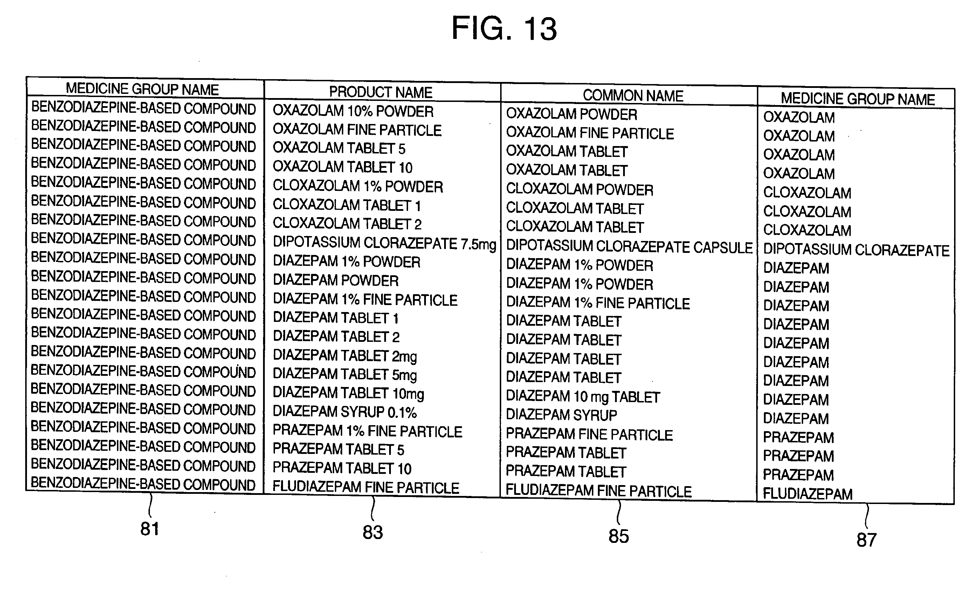

Further, for example, as shown in FIG. 13, a database which comprises medicine group names, product names, common names and component names may be disposed in the memory section 13. This database is constituted as a table in which a plurality of medicine product names 83 included in a predetermined medicine group name 81, and a common name 85 and a component name 87 corresponding to the product name are correlated.

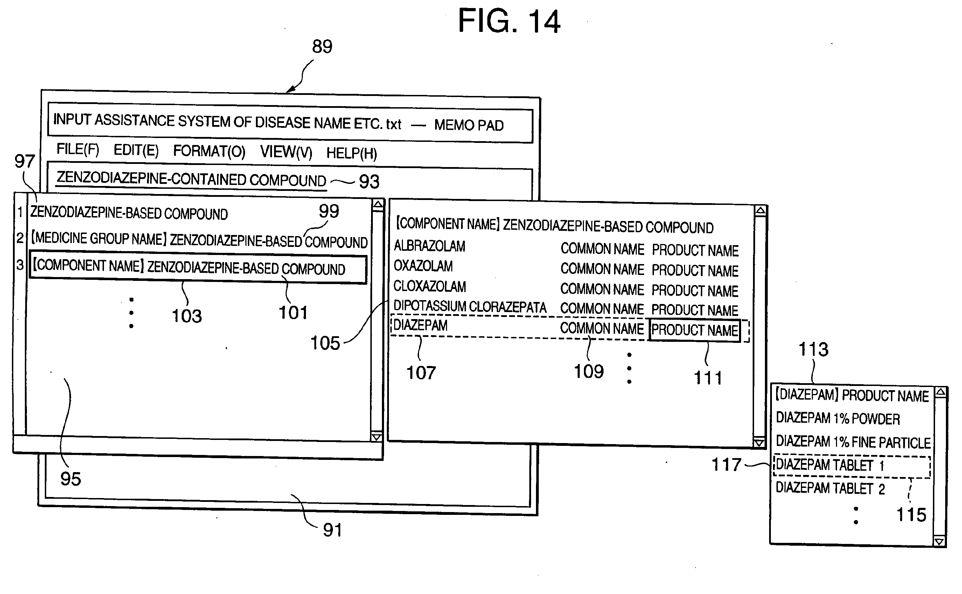

Now, description will be made as to an example of an operation of the character string input assistance program of the embodiment. FIG. 14 is a view showing an example of screen displaying of the character string input assistance program of the embodiment.

As shown in FIG. 14, on a screen display 89, there is disposed a character string input column 91 to which character string information is input. Then, when the user inputs a character string 93 to be converted to the character string input column 91 through the input section 5, and further inputs a conversion command, a list 95 is displayed which is a first layer of a list of conversion candidate databases each having a hierarchical structure. For example, if the character string 93 to be converted is "zenzodiazepine-based compound", the list 95 includes, e.g., a display 97 of a character string "zenzodiazepine-based compound" which is a conversion candidate character string retrieved from a general dictionary, a display 99 of "(medicine name) zenzodiazepine-based compound" indicating a location of a medicine group name DB related to the zenzodiazepine-based compound, and a display 101 of "(component name) zenzodiazepine-based compound" indicating a location of a component name DB related to the zenzodiazepine-based compound.

Then, when the user operates a cursor 103 to select, e.g., the display 101, for example, a list 105 indicating component names of the zenzodiazepine-based compound is displayed in an area of a screen display adjacent to the list 95. In this list, component names 107 are enumerated and, in each component name 107, a selection operation section 109 is displayed as a GUI to select a common medicine name and a product name of a relevant component. For example, when the user moves a cursor 111 to "product name" of a component name "diazepam" to input a selection command, a list 113 of medicine product names regarding the component "diazepam" is displayed in an area adjacent to the list 105. Then, the user moves a cursor 115 to superimpose it on a display 117 of an optional product name, and selects the product name, whereby the product name is input as an established character string to the character string input column 91.

The lists 95, 105 and 113 are properly prepared based on the database which comprises medicine codes and medicine group names, the database which comprises medicine group names and components, and the database which comprises medicine group names, product names and common names. Thus, according to the embodiment, by hierarchizing the database of conversion candidate character strings, it is possible to facilitate input of character strings even if there are many conversion candidate character strings.

For example, in order to reduce the load on the apparatus during normal document preparation which is not for any particular medical purposes, the function of the character string input assistance program may be constituted to be switched between validity and invalidity. In this case, for example, a button may be displayed in the input method status bar to switch the character string input assistance program between validity and invalidity.

According to the embodiment, the character string conversion assistance program is executed by the computer. However, the invention is not limited to this. For example, a medical electronic device, a cellular phone terminal (PDA), a cellular phone or the like which comprises a character string input function may be used.

According to the embodiment, the character string input assistance program is installed in the so-called stand-alone computer. Instead, however, a so-called application service provider system may be employed, in which the character string input assistance program is installed in, e.g., a server connected to a network such as Internet, and the user connects his own terminal to the network in order to use the program.

Furthermore, according to the embodiment, the conversion candidate DB is prepared beforehand in the memory section of the computer. However, this conversion candidate DB may be prepared in the server connected to the network, and accessed to be used from user's terminal which comprises the character string input assistance program. If the conversion candidate DB is prepared on the user side, contents of the conversion candidate DB may be sequentially updated by information download services through, e.g., a package medium such as an optical disk, or the network.

## Claims

1. A character string input assistance program for supplying a desired conversion candidate character string selected in accordance with an input character string to another program, comprising:
a step of receiving an input unconverted character string;
a step of receiving a conversion command for the unconverted character string;
a step of executing display for prompting selection of a plurality of conversion candidate databases constituted by pre-storing conversion candidate character strings in accordance with the conversion command;
a step of receiving a command for selecting a conversion candidate database;
a step of referring to the conversion candidate database selected in accordance with the command for selecting the conversion candidate database to present a conversion candidate character string included in the conversion candidate database;
a step of receiving a command for selecting a conversion candidate character string; and
a step of supplying the conversion candidate character string selected in accordance with the command for selecting the conversion candidate character string to the other predetermined program.

2. The character string input assistance program as claimed in Claim 1, wherein the other predetermined program is an electronic medical chart preparation program, and the plurality of conversion candidate databases include at least one selected from a disease name database, a disease name code database and a medicine name database.

3. The character string input assistance program as claimed in Claim 1, which is processed cooperatively with an input method program constituted independently of the other predetermined program.

4. The character string input assistance program as claimed in Claim 2, which is processed cooperatively with an input method program constituted independently of the other predetermined program.

5. A character string inputting apparatus for supplying a desired conversion candidate character string selected in accordance with an input character string to another program, comprising:
a memory section for storing a plurality of conversion candidate databases constituted by pre-storing conversion candidate character strings;
an information processing section connected to the memory section;
an input section for receiving an operation from a user; and
a display section for presenting information to the user; and
the apparatus having functions of executing display for prompting input of a command for selecting a conversion candidate database on a display section during execution of the other predetermined program in accordance with inputs of an unconverted character string and a conversion command, referring to the conversion candidate database selected in accordance with the input of the command for selecting the conversion candidate database to execute, on the display section, display for prompting input of a command for selecting a conversion candidate character string included in the conversion candidate database, and supplying the conversion candidate character string selected in accordance with the input of the command for selecting the conversion candidate character string to the other predetermined program.

6. A character string inputting method for supplying a desired conversion candidate character string selected in accordance with an input character string to another program, comprising:
a step of receiving an input unconverted character string;
a step of receiving a conversion command for the unconverted character string;
a step of executing display for prompting selection of a plurality of conversion candidate databases constituted by pre-storing conversion candidate character strings in accordance with the conversion command;
a step of receiving a command for selecting a conversion candidate database;
a step of referring to the conversion candidate database selected in accordance with the command for selecting the conversion candidate database to present a conversion candidate character string included in the conversion candidate database;
a step of receiving a command for selecting a conversion candidate character string; and
a step of supplying the conversion candidate character string selected in accordance with the command for selecting the conversion candidate character string to the other predetermined program.
